# EUROPEAN PATENT APPLICATION

(11) **EP 2 030 980 A1**
(43) Date of publication of application: **04.03.2009**
(21) Application number: 07115137.7
(22) Date of filing: 28.08.2007
(51) Int. Cl.: C07K 4/12

(54) **Mutants of lactoferrin**

(71) Applicant: AM-Pharma B.V., 3981 AK Bunnik (NL)
(72) Inventor: Brouwer, Cornelis Peter Johannes Maria, 3962 KX, Wijk bij Duurstede (NL); Jonk, Luigi Johannes Cornelius, 3571 BC, Utrecht (NL); Velders, Markwin Paul, 3721 BJ, Bilthoven (NL); Wulferink, Marty Bernardus Fransiscus, 6717 JE, Ede (NL)
(74) Representative: Hatzmann, Martin

(57) **Abstract**

The invention relates to field of biochemistry, molecular biology as well as medicinal chemistry. More in specific, the invention relates to polypeptides derived from human lactoferrin and their use in therapeutic or prophylactic treatment.

The invention provides a polypeptide comprising a mutant of the amino acid sequence RRRRSVQWC, which mutation comprising polypeptide has comparable antimicrobial activity against at least one micro-organism if compared to a reference polypeptide comprising the amino acid sequence RRRRSVQWC.

## Description

The invention relates to field of biochemistry, molecular biology as well as medicinal chemistry. More in specific, the invention relates to polypeptides derived from human lactoferrin and their use in therapeutic or prophylactic treatment.

Antimicrobial peptides have received increasing attention as a new pharmaceutical substance for combating infectious diseases. Often these antimicrobial peptides display a broad spectrum of antimicrobial activities which renders potentially useful in combating multi-drug-resistant pathogenic micro-organisms that have rapidly spread in recent years. Useful antimicrobial peptides have been found as endogenous peptides in mammals, birds, amphibia, insects, and plants where they appear to be components of the host defense systems. Frequently such endogenous antimicrobial peptides are cationic amphipathic molecules that contain 10 to 45 amino acid residues and an excess of lysine and arginine residues. Examples of cationic peptides include rabbit defensin, crab tachyplesin, bovine bactenecin, silk-moth cecropin A, frog magainins, bovine indolicidin. Yet another example is a peptide consisting of the first 11 amino acids of human lactoferrin (hLF1-11) which was reported to have broad spectrum antimicrobial activity against a wide variety of bacteria, fungi, viruses and other microbes (WO 01/34641).

Lactoferrin (LF) is a metal binding glycoprotein of Mr 77, 000 found in milk, tears, saliva, bronchial, intestinal, and other secretions. LF is also present in thesecolldal-y granules of neutrophils. Lactoferrin plays an important role in numerous inflammatory and immune response functions such as regulation of monocyte colony stimulating factor synthesis, activation of natural killer cell activity, inhibition of metastasis, and maturation of T-cells. Lactoferrin also inhibits myelopoiesis, binds to members of the low density lipoprotein receptor family, and blocks the clearance of lipoprotein chylomicron remnant particles. It also appears to play a role in inhibiting the production or release of prostaglandin E2, interleukins, and tumor necrosis factor by mononuclear cells.

Human lactoferrin (hLF) is also a major component of the non-specific defence of mucosal surfaces and neutrophils and is active against a variety of pathogens. This protein displays antimicrobial properties against Gram-positive and Gram-negative bacteria by limiting the availability of environmental iron. However, since iron-saturated hLF is also able to kill certain bacteria, mechanisms other than iron depletion apparently are involved in the antibacterial activity of lactoferrin.

The amino acid sequence of LF has been determined by protein sequencing and sequencing of cDNA clones. Human LF (hLF) consists of a polypeptide chain of 692 amino acids and contains two N-terminal cationic domains, i. e., RRRR (residues 2-5) and RKVR (residues 28-31), whereas bovine lactoferrin (bLF) has only one cationic domain (residues 17-42). The LF polypeptide is folded into two globular lobes, each of which contains an iron-binding cleft. The high affinity of LF for iron confers to the protein certain antibacterial properties and, in addition, may play a role in the absorption of dietary iron by the small intestine.

It has been reported that peptides of bLF origin as well as synthetic peptides that include the second cationic domain of hLF as well as peptides that include the first cationic domain of hLF show antibacterial activity resulting from depolarization of the membrane, increased membrane permeability and metabolic injury.

To increase the amount of available antimicrobial agents, the inventors of the present invention provide novel antimicrobial agents which can be used alone or in combination with each other or in combination with a conventional antimicrobial agent in combating a microbe.

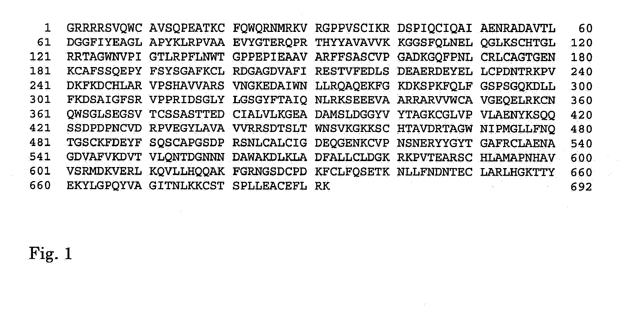

The novel antimicrobial agents of the invention are derived from human lactoferrin, more in specific from the first cationic cluster of human lactoferrin. The sequence of human lactoferrin is shown in Figure 1 in which residues 2-5 comprise the first cationic domain. Within the experimental part it is disclosed that numerous mutants of hLF1-11 (i.e. Gly-Arg-Arg-Arg-Arg-Ser-Val-Gln-Trp-Cys-Ala or G₁R₂R₃R₄R₅S₆V₇Q₈W₉C₁₀A₁₁) and more in specific numerous mutants of hLF2-10 (i.e. Arg-Arg-Arg-Arg-Ser-Val-Gln-Trp-Cys or R₂R₃R₄R₅S₆V₇Q₈W₉C₁₀) provide acceptable antimicrobial activity.

In a first embodiment, the invention provides a polypeptide comprising a mutant of the amino acid sequence RRRRSVQWC, which mutation comprising polypeptide has comparable antimicrobial activity against at least one micro-organism if compared to a reference polypeptide comprising the amino acid sequence RRRRSVQWC and wherein said mutant is:
- a deletion mutant in which at least one of the amino acids S, V, Q or W is deleted;
- a deletion mutant in which one R and at least one of the amino acids S, V, Q or W is deleted;
- a substitution mutant in which S, V or Q are substituted with a conservative or non-conservative amino acid, provided that (i) the substitute is not a negatively charged amino acid and (ii) S is not substituted with an N;
- a double substitution mutant in which SV, VQ or SQ are substituted with conservative or non-conservative amino acids, provided that none of the substitutes is a negatively charged amino acid;
- a triple substitution mutant in which SVQ are substituted with conservative or non-conservative amino acids, provided that none of the substitutes is a negatively charged amino acid;
- a substitution mutant in which W is replaced by another aromatic amino acid;
- a double substitution mutant in which Q or S is substituted by an aromatic amino acid and W is substituted by a neutral amino acid;
- a scrambled mutant in which C is switched with an amino acid in the SVQW sequence;
- a substitution mutant in which at least one but no more than three of the R's are substituted by another positively charged amino acid;
- a substitution mutant in which the second, third or fourth R is substituted by an A; or
- any combination thereof.

Herein, single-letter symbols are used to denote the amino acids, while three-letter symbols are used in the corresponding sequence listing. These symbols, which are well known to the person skilled in the art, have the following meaning: A = Ala = alanine, C = Cys = cysteine, D = Asp = aspartic acid, E = Glu = glutamic acid, F = Phe = phenylalanine, G= Gly = glycine, H= His = histidine, I=Ile = isoleucine, K = Lys = lysine, L = Leu = leucine, M= Met = methionine, N = Asn = asparagine, P = Pro = proline, Q = Gln = glutamine, R = Arg = arginine, S = Ser = serine, T = Thr = threonine, V = Val = valine, W = Trp = tryptophan, Y = Tyr = Tyrosine and X = Xaa = a variable amino acid.

Amino acids are typically divided into subgroups based on their properties. The following subgroups are typically used: hydrophobic (F, Y, W, H, K, T, C, A, G, A, V, L, M), polar (Y, W, H, K, R, T, C, S, N, Q, D, E), small (C, G, A, S, V, T, D, N, P), aliphatic (I, V, L), charged (H, K, R, D, E), aromatic or stacking (F, Y, W, H), negative (D, E), C-beta branched (V, I, T) and positive (H, K, R).

The terms "the amino acid sequence R₂R₃R₄R₅S₆V₇Q₈W₉C₁₀" and the "the amino acid sequence RRRRSVQWC" are used interchangeably herein and both refer to a sequence as naturally present in natural human lactoferrin. In case of the term "the amino acid sequence R₂R₃R₄R₅S₆V₇Q₈W₉C₁₀", the used numbers refer to the natural position of these amino acids in human lactoferrin. This does not mean that a polypeptide of the invention must comprise a mutant of the amino acid sequence RRRRSVQWC at positions 2 to 10. Furthermore, this does not mean, although not excluded, that the mentioned amino acid sequence is by definition part of a polypeptide comprising human lactoferrin. A polypeptide according to the invention thus comprises a mutated version of the amino acid sequence 2 to 10 of Figure 1.

Besides the particularly mentioned mutations a polypeptide according to the invention has a certain acceptable antimicrobial activity against at least one micro-organism. Whether or not an antimicrobial activity is considered acceptable is determined by comparing a polypeptide comprising a mutated RRRRSVQWC amino acid sequence with the antimicrobial activity of a polypeptide comprising a non-mutated RRRRSVQWC amino acid sequence. Although considered to be superfluous the inventors would like to point out that the sequences, if present, at the N-terminus and the C-terminus of RRRRSVQWC are thus identical in the mutated and non-mutated version of the polypeptide, i.e. a true comparison is made.

To compare antimicrobial activity of the mutants, the inventors made use of the minimal inhibitory concentration (MIC) assay, extensively described in the literature and which has become a world wide standardized test for e.g. determining susceptibility of strains towards antibiotics (reference "National Committee for Clinical Laboratory Standards: Performance standards for antimicrobial susceptibility testing; fourteenth informational supplement. M100-S14, Volume 24, Wayne, PA; 2004."). The MIC assay is a fast, reliable assay that is used throughout the world in medical microbiology divisions to test e.g. antibiotic response of clinical isolates to monitor antibiotic therapy. MIC values represent the lowest concentration of antibiotic or antifungal that inhibits >90% of growth of the micro-organism. In hospitals MIC values are also used to determine strength of antibiotic therapy where one of the pharmacokinetic parameters is expressed as "hours over MIC". The inventors therefore feel that MIC values, in contrast to e.g. killing assays, more closely relate to clinical efficacy of the drug and predict more correctly the success of a drug in the clinic.

A mutant of the amino acid sequence RRRRSVQWC is for example a mutant in which at least one amino acid is deleted or wherein at least one amino acid is substituted (or replaced) by another amino acid. It is clear from the experimental part that a deletion or substitution can also involve two, three, four or even more amino acids of RRRRSVQWC. The next sections provide example of suitable mutants.

One example of a mutant is a deletion mutant in which at least one of the amino acids S, V, Q or W is deleted, such as RRRRVQWC, RRRRSQWC, RRRRSVWC or RRRRSVQC. As shown in the experimental part herein, a deletion mutant in which at least two of the amino acids S, V, Q and/or W are deleted also have an acceptable antimicrobial activity: RRRRQWC, RRRRSWC or RRRRSVC. Double deletion mutants can also comprise mutants in which one R and at least one of the amino acids S, V, Q or W are deleted: RRRVQWC, RRRSQWC, RRRSVWC or RRRSVQC. A deletion mutant can also comprise the sequence RRRQWC, RRRRWC or RRRRSC, i.e. a deletion mutant in which 3 amino acids have been deleted. Yet another example is a deletion mutant in which 4 amino acids have been deleted, i.e. RRRRC.

In a preferred embodiment, a deletion mutant is a deletion mutant in which S, V, Q or W is deleted or a deletion mutant in which RS, SV, VQ or QW are deleted. Thus, preferably a polypeptide according to the invention comprises RRRRVQWC, RRRRSQWC, RRRRSVWC, RRRRSVQC, RRRVQWC, RRRRQWC, RRRRSWC or RRRRSVC.

Another example of a mutant is a substitution mutant in which S, V or Q are substituted with a conservative or non-conservative amino acid (i.e. any amino acid), provided that (i) the substitute is not a negatively charged amino acid and (ii) S is not substituted with an N. A substitution by a conservative amino acid involves a change of amino acid within one of the above described groups, for example a hydrophobic amino acid by another hydrophobic amino acid or a positively charged amino acid by another positively charged amino acid. A substitution by a non-conservative amino acid involves an amino acid of another group, for example a positively charged amino acid by a negatively charged amino acid. In other words, the invention also provides a polypeptide comprising the amino acid sequence:
RRRRXbbVQWC in which Xbb is A, C, F, G, H, I, K, L, M, P, Q, R, T, V, W or Y; or
RRRRSXccQWC in which Xcc is A, C, F, G, H, I, K, L, M, N, P, Q, R, S, T, W or Y; or
RRRRSVXjjWC in which Xjj is A, C, F, G, H, I, K, L, M, N, P, R, S, T, V, W or Y.

A further example of a mutant is a double substitution mutant in which SV, VQ or SQ are substituted with conservative or non-conservative amino acids, provided that none of the substitutes is a negatively charged amino acid. In other words, the invention provides a polypeptide comprising the amino acid sequence: RRRRXddXccQWC or RRRRSXccXjjWC or RRRRXddVXjjWC in which
Xdd is selected from A, C, F, G, H, I, K, L, M, N, P, Q, R, T, V, W or Y;
Xcc and Xjj are as identified above.
Especially preferred is a polypeptide comprising a mutant of the RRRRSVQWC sequence, wherein said mutant is a double substitution mutant in which SV or VQ are substituted with conservative or non-conservative amino acids, provided that none of the substitutes is a negatively charged amino acid, i.e. a polypeptide comprising the amino acid sequence RRRRXddXccQWC or RRRRSXccXjjWC and wherein Xdd, Xcc and Xjj are as identified above.

As yet another example, the invention also provides a polypeptide comprising a mutant of the amino acid sequence RRRRSVQWC, which mutation comprising polypeptide has comparable antimicrobial activity against at least one micro-organism if compared to a reference polypeptide comprising the amino acid sequence RRRRSVQWC and wherein said mutant is a triple substitution mutant in which SVQ are substituted with conservative or non-conservative amino acids, provided that none of the substitutes is a negatively charged amino acid, i.e. a mutant with the amino acid sequence RRRRXddXccXjjWC and wherein Xdd, Xcc and Xjj are as identified above.

The mutation can not only reside in the SVQ part of the amino acid sequence RRRRSVQWC, but can also involve the W. One example of a corresponding mutant is a substitution mutant in which W is replaced by another aromatic amino acid. Examples of suitable amino acids are F, Y or H. This results in a polypeptide comprising the sequence RRRRSVQFC, RRRRSVQYC, or RRRRSVQHC. In a preferred embodiment, a polypeptide according to the invention comprises the sequence RRRRSVQFC, i.e. a mutant in which the W has been replaced by an F.

Another mutation that involves the W, is a double substitution mutant in which Q or S is substituted by an aromatic amino acid and W is substituted by a neutral amino acid. This results in a polypeptide comprising the amino acid sequence RRRRSVXggXiiC or RRRRXggVQXiiC, wherein Xgg is F, Y, H or W and wherein Xii is T, C, S, N or Q. In a preferred embodiment, Xgg is an F and/or Xii is T.

When the original W (i.e. position 9 of R₂R₃R₄R₅S₆V₇Q₈W₉C₁₀) is replaced by an S or Q and the original S or Q (i.e. position 6 or 8 of R₂R₃R₄R₅S₆V₇Q₈W₉C₁₀) is replaced by a W, the aromatic amino acid (W) has actually changed places with a neutral amino acid. Yet a specific example of a mutation that involves the W is a mutant in which W has changed places with S or Q.

In yet another embodiment, said mutant is a double substitution mutant in which V substituted by an aromatic amino acid and W is substituted by a neutral amino acid. This results in a polypeptide comprising the amino acid sequence RRRRSXggQXiiC, wherein Xgg is F, Y, H or W and wherein Xii is T, C, S, N or Q. In yet another embodiment, the aromatic acid W has changed places with V: RRRRSWQVC.

A further part of the RRRRSVQWC sequence that can be modified is the C. This amino acid is important for the activity of the resulting polypeptide and may not be deleted. However, the C can switch places within the SVQWC sequence essentially without compromising the antimicrobial activity. The invention therefore also provides a polypeptide comprising a mutant of the amino acid sequence RRRRSVQWC, which mutation comprising polypeptide has comparable antimicrobial activity against at least one micro-organism if compared to a reference polypeptide comprising the amino acid sequence RRRRSVQWC and wherein said mutant is a scrambled mutant in which C is switched with an amino acid in the SVQW sequence.
Examples are RRRRSVQCW, RRRRSVCWQ, RRRRSCQWV or RRRRCVQWS. A preferred mutant is RRRRCVQWS, i.e. a scrambled mutant in which S and C have changed places.

Another region in the amino acid sequence RRRRSVQWC that can be mutated is the RRRR part thereof. An example is a substitution mutant in which at least one but no more than three of the R's are substituted by another positively charged amino acid, such as H or K. Preferably said another charged amino acid is K. This results in a polypeptide according to the invention comprising a mutant of the amino acid sequence RRRRSVQWC, wherein said mutant is (H or K)RRRSVQWC or R(H or K)RRSVQWC or RR(H or K)RSVQWC or RRR(H or K)SVQWC.

A further example of a mutant in the RRRR region of RRRRSVQWC is a double substitution mutant in which two of the four R's are substituted by other positively charged amino acids, such as H or K. The resulting polypeptide comprises the amino acid sequence (H or K)(H or K)RRSVQWC, (H or K)R(H or K)RSVQWC, (H or K)RR(H or K)SVQWC, R(H or K)(H or K)RSVQWC, R(H or K)R(H or K)SVQWC or RR(H or K)(H or K)SVQWC. In a preferred embodiment the mutant is KKRRSVQWC, RKKRSVQWC or RRKKSVQWC, i.e. a polypeptide wherein said mutant is a double substitution mutant in which the first and second, second and third, or the third and the fourth R are substituted by KK.

Another example of a mutant within the RRRR region is a substitution mutant in which the second, third or fourth R is substituted by an A, thus RARRSVQWC, RRARSVQWC or RRRASVQWC.

Based on the described mutants, a skilled person can also prepare a polypeptide comprising a combination of the described mutants such as but not limited to a deletion of S in combination with a replacement of W by another aromatic amino acid such as F, Y or H.

The invention not only provides a polypeptide comprising a mutant of the amino acid sequence RRRRSVQWC, which mutation comprising polypeptide has comparable antimicrobial activity against at least one micro-organism if compared to a reference polypeptide comprising the amino acid sequence RRRRSVQWC and wherein said mutant is a deletion or substitution mutant or a combination thereof, but said mutant can also be a insertion mutation, i.e. amino acids can be added to the N- or C-terminus of the amino acid sequence RRRRSVQWC and can equally well be added internally in the RRRRSVQWC amino acid sequence.

The mutated RRRRSVQWC sequence can be linear as well as cyclic. In a preferred embodiment, the invention provides a polypeptide comprising a mutant of the amino acid sequence RRRRSVQWC, which mutation comprising polypeptide has comparable antimicrobial activity against at least one micro-organism if compared to a reference polypeptide comprising the amino acid sequence RRRRSVQWC and wherein said mutant is as described above and wherein the mutated amino acid sequence RRRRSVQWC is linear.

As described above, the mutated RRRRSVQWC amino acid sequence can be part of a (somewhat) bigger polypeptide, such as, but not limited to a polypeptide that has N terminally been extended by a G and/or C-terminally been extended by an A, leading to a mutated GRRRRSVQWCA amino acid sequence in which any of the above described mutation is introduced. The length of the final polypeptide has a length of up to a 1000 amino acids or more, preferably the polypeptide has a length of 5 to 700 amino acids.

To keep the production costs as low as possible it is preferred to keep the polypeptide as small as possible. In a preferred embodiment, a polypeptide according to the invention has a length of 5, 6, 7 or 8 amino acids. In yet another preferred embodiment, a polypeptide according to the invention has a length of 9, 10 or 11 or up to 18 amino acids.
Examples of polypeptides with different lengths are provided above as well as in the experimental part.

The polypeptides according to the invention can be prepared via different methods and means.

Particularly because some of the polypeptides of the invention are relatively short, the polypeptides can be readily synthesized using known methods. For example, the polypeptides can be synthesized by the well-known Merrifield solid-phase synthesis method in which amino acids are sequentially added to a growing chain. See Merrifield (1963), J. Am. Chem. Soc. 85: 2149-2156 ; and Atherton et al.,"Solid Phase Peptide Synthesis,"IRL Press, London, (1989). Automatic peptide synthesizers are commercially available from numerous suppliers, such as Applied Biosystems, Foster City, California.

Alternatively, the polypeptides of the invention can be prepared using well known recombinant techniques in which a nucleotide sequence encoding the polypeptide of interest is expressed in cultured cells such as described in Ausubel et al., Current Protocols in Molecular Biology, Greene Publishing and Wiley-Interscience, New York (1987) and in Sambrook et al., Molecular Cloning-A Laboratory Manual, 2nd ed., vols. 1-3, Cold Spring Harbor Laboratory, Cold Spring Harbor, NewYork, 1989, both of which are incorporated herein by reference in their entirety. Also see, Kunkel, 1985, Proc. Natl. Acad. Sci. 82: 488 (describing site directed mutagenesis) and Roberts et al., 1987, Nature 328: 731-734 or Wells, J.A., et al. (1985) Gene 34 :315 (describing cassette mutagenesis).

Typically, nucleic acids encoding the desired polypeptides are used in expression vectors. The phrase "expression vector" generally refers to nucleotide sequences that are capable of affecting expression of a gene in hosts compatible with such sequences.

These expression vectors typically include at least suitable promoter sequences and optionally, transcription termination signals. Additional factors necessary or helpful in effecting expression may also be used as described herein. DNA encoding the polypeptides of the present invention will typically be incorporated into DNA constructs capable of introduction into and expression in an in vitro cell culture. Specifically, DNA constructs will be suitable for replication in a prokaryotic host, such as bacteria, e.g., E. coli, or may be introduced into a cultured mammalian, plant, insect, yeast, fungi or other eukaryotic cell lines.

DNA constructs prepared for introduction into a particular host will typically include a replication system recognized by the host, the intended DNA segment encoding the desired polypeptide, and transcriptional and translational initiation and termination regulatory sequences operably linked to the polypeptide encoding segment. A DNA segment is "operably linked" when it is placed into a functional relationship with another DNA segment.

For example, a promoter or enhancer is operably linked to a coding sequence if it stimulates the transcription of the sequence. DNA for a signal sequence is operably linked to DNA encodinga polypeptide if it is expressed as a preprotein that participates in the secretion of the polypeptide. Generally, DNA sequences that are operably linked are contiguous, and, in the case of a signal sequence, both contiguous and in reading phase. However, enhancers need not be contiguous with the coding sequences whose transcription they control. Linking is accomplished by ligation at convenient restriction sites or at adapters or linkers inserted in lieu thereof.

The selection of an appropriate promoter sequence generally depends upon the host cell selected for the expression of the DNA segment. Examples of suitable promoter sequences include prokaryotic, and eukaryotic promoters well-known in the art. See, e.g, J., Sambrook et al., Molecular Cloning : A Laboratory Manual (2nd ed.), vols. 1-3, Cold Spring Harbor Laboratory (1989). The transcriptional regulatory sequences will typically include a heterologous enhancer or promoter which is recognized by the host. The selection of an appropriate promoter will depend upon the host, but promoters such as the trp, lac and phage promoters, tRNA promoters and glycolytic enzyme promoters are known and available. See Sambrook et al., supra.

Conveniently available expression vectors which include the replication system and transcriptional and translational regulatory sequences together with the insertion site for the polypeptide encoding segment may be employed. Examples of workable combinations of cell lines and expression vectors are described in Sambrook et al., supra, and in Metzger et al., Nature 334 : 31-36 (1988). For example, suitable expression vectors may be expressed in, e. g., insect cells, e.g., Sf9 cells, mammalian cells, e. g., CHO cells and bacterial cells, e. g., E. coli.

In certain instances, the polypeptides of the invention are produced by expression in transgenic animals (i.e., non-human animals containing an exogenous DNA sequence in the genome of germ-line and somatic cells introduced by way of human intervention) such as bovines, goats, rabbits, sheep, pigs or mice. Methods for production of recombinant polypeptides by transgenic nonhuman species are known in the art and are described, for example, in U. S. Patent Nos. 5, 304, 489; 5, 633, 076; and 5, 565, 362 which are incorporated herein by reference in their entirety, as well as in PCT publications PCT/US93/05724 and PCT/US95/09580, both of which are incorporated herein by reference in their entirety. An advantage of the transgenic animals is the isolation of the polypeptides of interest in large amounts, especially by economical purification methods. For example, the production of transgenic bovine species containing a transgene encoding a human lactoferrin polypeptide targeted for expression in mammary secreting cells is described in WO 91/08216, incorporated herein by reference in its entirety. When lactoferrin variants are produced in transgenic bovines the human protein typically is separated from the bovine milk proteins (e. g., whey proteins, caseins, bovine lactoferrin, IgA, albumin, lysozyme, ss-lactoglobulin) before use (e. g., administration to patients). Alternatively, use may be made of whole or partially purified bovine milk containing the desired polypeptide.

Another method for preparing the polypeptides of the invention is to employ an in vitro transcription/translation system. DNA encoding a polypeptide of the invention is cloned into an expression vector as described supra. The expression vector is then transcribed and translated in vitro. The translation product can be used directly or first purified. Polypeptides resulting from in vitro translation typically do not contain the posttranslation modifications present on polypeptides synthesized in vivo. Methods for synthesis of polypeptides by in vitro translation are described by, for example, Berger & Kimmel, Methods in Enzymology, Volume 152, Guide to molecular Cloning Techniques, Academic Press, Inc., San Diego, CA, 1987 (incorporated herein by reference in its entirety).

In yet another embodiment, the invention therefore also provides a nucleic acid encoding any of the herein provided polypeptides, as well as vector comprising said nucleic acid. The invention further provides a host cell comprising said nucleic acid or vector.

The invention further provides an antibody which specifically binds to any of the polypeptides of the invention. Monoclonal antibodies are made from the polypeptides of the invention or from antigen-containing fragments thereof by methods that are well-known in the art (see, e. g., Kohler, et al. Nature, 256: 495, (1975); and Harlow & Lane, Antibodies, A Laboratory Manual (C. S. H. P., NY, 1988), both of which are incorporated herein by reference in their entirety). The antibodies of the invention are useful in purifying polypeptides of the invention, in screening cDNA expression libraries, and for identifying clones containing cDNA inserts which encode structurally-related, immunocrossreactive polypeptides.

The above described polypeptides can exclusively consist of any of the above described mutant sequences or the described mutant sequences are N- and/or C-terminally extended. One example of an extended polypeptide is one in which a G has been added to the N-terminus and/or in which an A has been added to the C-terminus, for example GRRRSVQWCA in which the core sequence RRRRSVQWC has been mutated as herein described. Yet another example is a polypeptide selected from the group of:
(a) a polypeptide comprising 8, 9, 10, 11 or 12 but no more than 18 contiguous amino acids from the human lactoferrin protein (Figure 1), wherein the N-terminus of said protein is residue 1 of Figure 1;
(b) a polypeptide comprising at least 7 but no more than 27 contiguous amino acids from the human lactoferrin protein (Figure 1), wherein the N-terminus of said polypeptide is residue 2 of Figure 1
and wherein the sequence RRRRSVQWC has been mutated as herein described.

Furthermore or additionally a polypeptide according to the invention can be provided with a second moiety. Examples of a suitable second moiety will be provided below. All described second moieties can also be coupled to non-mutated hLF1-11, i.e. coupled to GRRRRSVQWCA.

In a preferred embodiment, said second moiety is a protein which can be directly coupled to a polypeptide of the invention or indirectly via a spacer.
One example of a second moiety is biotin or fluorescein.

A further example of a second moiety is a targeting moiety which recognizes a target microbial organism. Examples of a targeting moiety are provided in US 2004/0137482 and include a polypeptide or small peptide and is fused in frame with a polypeptide according to the invention, i.e. a polypeptide comprising a mutant of the amino acid sequence RRRRSVQWC, which mutation comprising polypeptide has comparable antimicrobial activity against at least one micro-organism if compared to a reference polypeptide comprising the amino acid sequence RRRRSVQWC and wherein said mutant is as described above. A composition of a polypeptide comprising a mutant of the amino acid sequence RRRRSVQWC together which a proteinaceous second moiety can be produced recombinantly according to any one of the earlier described methods. Other examples of a second moiety are a ligand, receptor, antibody, that specifically interact with a target microbial organism.

In yet another embodiment, the second moiety is a hormone, such as a pheromone. A suitable example of a pheromone is the natural bacterial pheromone CSP which is especially useful in combating Streptococcus mutans. Such a pheromone or a functional fragment (especially the C-terminal 16 amino acids) thereof is coupled to any of the above described mutated RRRRSVQWC comprising polypeptide directly or via a linker.

Such a biotin, fluorescein, hormone or pheromone can further be coupled to non-mutated hLF1-11, i.e. coupled to GRRRRSVQWCA.

In a further embodiment, the second moiety is a sugar group, for example a mannose.

The mannose can be coupled to the peptide using a chemical reaction which is described in detail in example I. The chemical reaction is based on the activation of mannonic acid using N-(3-Dimethylaminopropyl-N'-ethylcarbodiimide hydrochloride (EDAC).

In a preferred embodiment, said mannose is coupled to said polypeptide via a chemical coupling. Such a sugar group and in specific such a mannose group can also be coupled to non-mutated hLF1-11, i.e. coupled to GRRRRSVQWCA.

In yet another embodiment, said second moiety is a lipid. However, such a lipid can also be coupled to non-mutated hLF1-11, i.e. coupled to GRRRRSVQWCA. Examples of suitable lipids involve the addition of acyl chains of 4, 8 or 12 C atoms between a lysine (Improved antimicrobial peptides based on acyl-lysine oligmers. I.S. Radzishevsky et al. Nature Biotechnology, 25(6): 657-659). The presence of such acyl side chains prevents the formation of stable secondary structures and improves the antimicrobial action.
However, acyl side chains can also be added to the N- and/or C-terminus of a a polypeptide according to the invention. In a preferred embodiment, one or more acyl chains of variable length, preferred: 4, 8 or 12 C atoms at the C or N terminus or at any other amino acid known to be dispensable for the antimicrobial activity can be added to a polypeptide as described herein. The acyl chain(s) can also be placed between two adjacent amino acids, preferably two arginines, to increase antimicrobial activity.

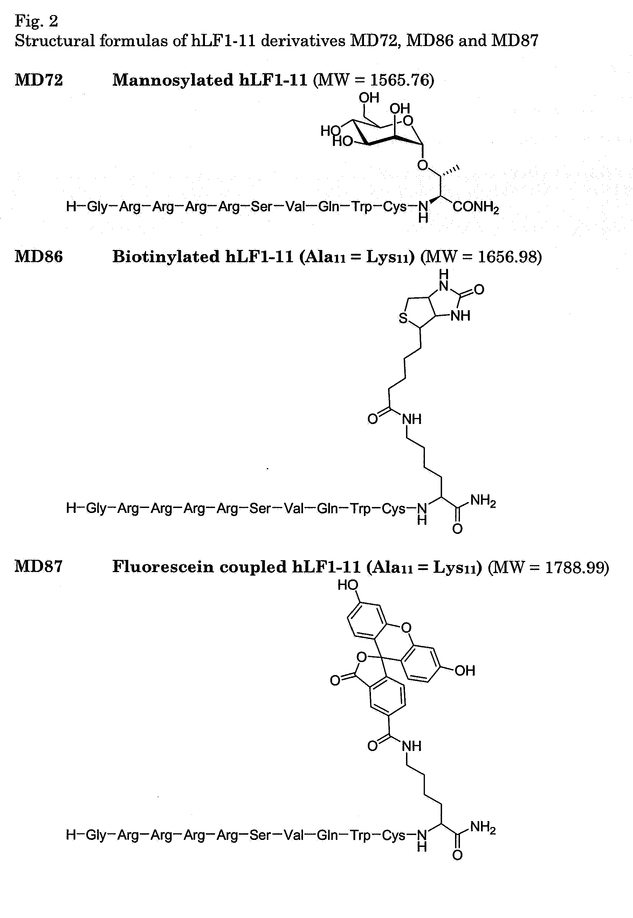

Examples of some the described second moieties are shown in Figure 2.

The polypeptides according to the invention, optionally coupled to a second moiety find their application in the field of pharmaceutics and more in specific in the treatment or prophylaxis of microbial infections.

The invention for example provides use of a polypeptide as described above for use as a medicament. The invention also provides a pharmaceutical composition comprising a polypeptide according to the invention and a pharmaceutical acceptable carrier, diluent or excipient. Although, the polypeptides according to the invention are considered to be potent antimicrobial agent, they can be combined with a conventional antimicrobial agent to increase or broaden the action of a pharmaceutical of the invention. In yet another embodiment, the invention thus provides a pharmaceutical according to the invention, further comprising a conventional antimicrobial agent.

The invention further provides use of a polypeptide as described herein for the manufacture of a medicament for treating a patient infected with a microbe or for the treatment or prophylaxis of microbial infections. In a preferred embodiment, said microbe is a bacterium, a fungus, a virus or a parasite.

The polypeptides and pharmaceutical compositions of the invention exhibit a number of biological activities that provide benefit in therapeutic or prophylactic applications. For example, as described in greater detail in the Examples below, the compositions are useful in treating various microbial infections such as bacterial infections.

The polypeptides and pharmaceutical compositions may also have various other beneficial activities. These include anti-inflammatory, anti-viral and anti-infective activities, as well a pro-and anti-coagulant effects, modulation of complement activation, inhibition lipopolysaccharide- (LPS) mediated activation of neutrophils, and growth promotion of intestinal epithelial cells. Other properties and biological activities of lactoferrin are described in Nuijens et al., 1996, J. Mammary Gland Biology and Neoplasia 1: 3, 283-293, which is incorporated herein by reference in its entirety.

Therapeutic indications for the pharmaceutical compositions described herein include use in therapy or prophylaxis of infection, including local infection, large scale (bacterial) infection, blood-borne infections, sepsis, as well as inflammation resulting from an infection or non-infectious inflammatory diseases (e.g. chronic inflammatory disease of the ileum or colon). The compositions can also be used to prepare or treat organ transplant recipients or other immunosuppressed individuals (e.g. AIDS patients) against the effects of infections.

The pharmaceutical compositions are effective in treating a variety of microbial infections, such as various viral, bacterial and fungal infections. For example, the compositions are effective in treating Gram-negative and Gram-positive bacteria. More specifically, some examples of pathogenic bacteria causing infections treatable by methods of the invention include: Listeria, Escherichia, chlamydia, rickettsial bacteria, mycobacteria, staphylococci, streptocci, pneumonococci, meningococci and conococci, Klebsiella, proteus, serratia, pseudomonas, Legionella, diphtheria, salmonella, bacilli, cholera, tetanus, botulism, anthrax, plague, leptospirosis, and Lymes disease bacteria.

Some examples of pathogenic viruses causing infections treatable by methods of the invention include: hepatitis (A, B or C), herpes virus (e. g., VZV, HSV-I, HAV-6, HSV-II, CMV, EpsteinBarr-virus), adenovirus, influenza virus, flaviviruses, echovirus, rhinovirus, coxsackie virus, coronavirus, respiratory syncytial virus (RSV), mumps virus, rotavirus, measles virus, rubella virus, parvovirus, vaccinia virus, HTLV virus, dengue virus, papillomavirus, molluscum virus, poliovirus, rabies virus, JC virus, arbo viralencephalitis virus, and human immunodeficiency virus (HIV virus; e. g., type I and II).

Some examples of pathogenic fungi causing infections treatable by methods of the invention include: Candida (e. g., albicans, krusei, glabrata, tropicalis), Cryptococcus neoformans, Aspergillus (e.g., fumigatus, niger), Genus Mucorales (Mucor, Absidia, Rhizopus), Sporothrix schenkii, Blastomyces dermatitidis, Paracoccidioides brasiliensis, Coccidioides immitis and Histoplasma capsulatum_{.}

Some examples of pathogenic parasites causing infections treatable by methods of the invention include: Entamoeba histolytica, Balantidium coli, Naegleria, Fowleri, Acanthamoeba sp., Giardia lambia, Cryptosporidium sp., Pneumocystis carinii, Plasmodiumvivax, Babesia microti, Trypanosoma brucei, Trypanosoma cruzi, Leishmania donovani, Toxoplasma gondii and Plasmodium falciparis.

The efficacy of the polypeptides and pharmaceutical compositions of the invention may be further improved by combining the use of the compositions of the invention with compositions for treating microbial infections known in the art per se. As such, the polypeptides and compositions of the invention may be combined with e. g. penicillins, cephalosporins, macrolides, fluoroquinolones, sulfonamides, tetracylcines or aminoglycosides for treating bacterial infections. For treating viral infections they may be combined with antiviral nucleoside analogs such as aciclovir, ganciclovir, zidovudine (AZT) or didanosine or neuramidase inhibitors such as oseltamivir, peramivir or zanamivir. Similarly, for treating fungal infections the polypeptides and compositions of the invention may be combined with polyene antifungals, imidazoles, triazoles, allylamines, echinocandins or others, such as ciclopirox, flucytosine, griseofulvin, gentian violet, haloprogin, tolnaftate or undecylenic acid.

The particular form of the composition varies with the intended mode of administration and therapeutic application. Typically, however, the composition includes a polypeptide and a pharmaceutically acceptable excipient, wherein the polypeptide is a mutant as described herein and has antimicrobial activity (e. g. is effective in killing viruses or bacteria). The polypeptides used in the pharmaceutical compositions can include any of the polypeptides described above.

The compositions may also include depending on the formulation desired pharmaceutically-acceptable, non-toxic carriers of diluents, which are defined as vehicles commonly used to formulate pharmaceutical compositions for animal or human administration. The diluent is selected so as not to affect the biological activity of the combination. Examples of such diluents are distilled water or water for injection, buffered water, physiological saline, PBS, Ringer's solution, dextrose solution, and Hank's solution. In addition, the pharmaceutical composition or formulation may also include other carriers, adjuvants, or non-toxic, nontherapeutic, nonimmunogenic stabilizers, excipients and the like. The compositions may also include additional substances to approximate physiological conditions, such as pH adjusting and buffering agents, toxicity adjusting agents, wetting agents, detergents and the like. Because of the ability of hLF to bind iron in some instances it may be beneficial to include iron in the pharmaceutical composition.

The composition may also include any of a variety of stabilizing agents, such as an antioxidant for example. Moreover, the polypeptides may be complexed with various well-known compounds that enhance the in vivo stability of the polypeptide, or otherwise enhance its pharmacological properties (e. g., increase the half-life of the polypeptide, reduce its toxicity, or enhance solubility or uptake). Examples of such modifications or complexing agents include the production of sulfate, gluconate, citrate, phosphate and the like. The polypeptides of the composition may also be complexed with molecules that enhance their in vivo attributes. A list of such molecules, provided by way of example and not limitation, includes carbohydrates, polyamines, amino acids, other peptides, ions (e. g., sodium, potassium, calcium, magnesium, manganese), and lipids.

Further guidance regarding formulations that are suitable for various types of administration can be found in Remington's Pharmaceutical Sciences, Mace Publishing Company, Philadelphia, PA, 17th ed. (1985). For a brief review of methods for drug delivery, see, Langer, Science 249: 1527-1533 (1990). Both of these references are incorporated herein by reference in their entirety.

The compositions containing the polypeptides can be administered for prophylactic and/or therapeutic treatments. The polypeptide in the pharmaceutical composition typically is present in a therapeutic amount, which is an amount sufficient to remedy a disease state or symptoms, particularly symptoms associated with a microbial infection, or otherwise prevent, hinder, retard, or reverse the progression of disease or infection or any other undesirable symptoms in any way whatsoever. The concentration of the polypeptide in the pharmaceutical composition can vary widely, i. e., from less than about 0.1% by weight, usually being at least about 1% by weight, to as much as 20% by weight or more.

In therapeutic applications, compositions are administered to a patient already suffering from a disease, as just described, in an amount sufficient to cure or at least partially arrest the symptoms of the disease or infection and its complications. An appropriate dosage of the pharmaceutical composition or polypeptide of the invention is readily determined according to any one of several well-established protocols. For example, animal studies (e. g., mice, rats) are commonly used to determine the maximal tolerable dose of the bioactive agent per kilogram of weight. In general, at least one of the animal species tested is mammalian.

The results from the animal studies can be extrapolated to determine doses for use in other species, such as humans for example.

What constitutes an effective dose also depends on the nature and severity of the disease or condition, and on the general state of the patient's health, but will generally range from about 0.1 µg to 10 mg of peptide per kilogram of body weight, with dosages of from about 0.5 to 500 µg per kilogram being more commonly employed. Especially suitable dosages are approximately 400 µg/kg for oral administration and approximately 4-80 µg/kg for intravenous administration. The mentioned ranges are especially suitable for a treatment based on a peptide of up to approximately 18, preferably 12, 11, 10, 9, 8, 7, 6 or 5 amino acids. If the second moiety coupled to a peptide is very large, changes need to be made to the indicated ranges. In prophylactic applications, compositions containing the compounds of the invention are administered to a patient susceptible to or otherwise at risk of a particular disease or infection. Such an amount is defined to be a "prophylactic effective" amount or dose. In this use, the precise amounts again depends on the patient's state of health and weight. Typically, the dose ranges from about 0.1 µg to 10 mg of peptide per kilogram of body weight, with dosages of from about 0.5 to 500 µg per kilogram being more commonly utilized. These dosages are typically daily dosages. The pharmaceutical compositions described herein can be administered in a variety of different ways. Illustrative examples include administering a composition containing a pharmaceutically acceptable carrier via oral, intranasal, rectal, topical, intraperitoneal, intravenous, intramuscular, subcutaneous, subdermal, transdermal, intrathecal, and intracranial methods. The preferred formulation and delivery option typically varies depending upon the location and size of the area requiring treatment. For example, for localized infections, the formulation may be designed for topical application or localized injection, for example. Systemic reactions, in contrast, may be treated or prevented by administration of compositions formulated for parenteral administration.

For oral administration, the active ingredient can be administered in solid dosage forms, such as capsules, tablets, and powders, or in liquid dosage forms, such as elixirs, syrups, and suspensions. The active component (s) can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that may be added to provide desirable color, taste, stability, buffering capacity, dispersion or other known desirable features are red iron oxide, silica gel, sodium lauryl sulfate, titanium dioxide, and edible white ink. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract.

Liquid dosage forms for oral administration can contain coloring and flavoring to increase patient acceptance.

If desired, for example in the treatment of infections or disorders of the digestive tract or even for general oral administration of the compositions, it is possible to formulate solid or liquid formulations in an enteric-coated or otherwise protected form. In the case of liquid formulations, the formulation can be mixed or simply co-administered with a protectant, such as a liquid mixture of medium chain triglycerides, or the formulation can be filled into enteric capsules (e. g., of soft or hard gelatin, which are themselves optionally additionally enteric coated). Alternatively, solid formulations comprising the polypeptide can be coated with enteric materials to form tablets. The thickness of enteric coating on tablets or capsules can be, for example, from 0. 5 to 4 microns in thickness. The enteric coating may comprise any of the enteric materials conventionally utilized in orally administrable pharmaceutical formulations. Suitable enteric coating materials are known, for example, from Remington's Pharmaceutical Sciences, Mace Publishing Company, Philadelphia, 17th ed. (1985) ; and Hagars Handbucher Pharmazeutischen Praxie, Springer Verlag,4th ed., Vol. 7a(1971), both of which are incorporated herein by reference in their entirety.

Another delivery option involves loading the composition into lipid-associated structures (e. g., liposomes, or other lipidic complexes) which may enhance the pharmaceutical characteristics of the polypeptide component of the composition. The complex containing the composition may subsequently be targeted to specific target cells by the incorporation of appropriate target molecules (c. g., specific antibodies or receptors). It is also possible to directly complex the polypeptide with a targeting agent.

Compositions prepared for intravenous administration typically contain 100 to 500ml of sterile 0. 9%NaCI or 5% glucose optionally supplemented with a 20% albumin solution and 10 µg to 1000 mg of a polypeptide of the invention. A typical pharmaceutical composition for intramuscular injection would be made up to contain, for example 1 ml of sterile buffered water and 10 µg to 10 mg of the purified polypeptide of the invention. Methods for preparing parenterally administrable compositions are well-known in the art and described in more detail in various sources, including, for example, Remington's Pharmaceutical Science, Mack Publishing, Philadelphia, PA, 17th ed., (1985) (previously incorporated herein by reference in its entirety).

Particularly when the compositions are to be used in vivo, the components used to formulate the pharmaceutical compositions of the present invention are preferably of high purity and are substantially free of potentially harmful contaminants (e. g., at least National Food (NF) grade, generally at least analytical grade, and more typically at least pharmaceutical grade). Moreover, compositions intended for in vivo use are usually sterile.

To the extent that a given compound must be synthesized prior to use, the resulting product is typically substantially free of any potentially toxic agents, particularly any endotoxins, which may be present during the synthesis or purification process. Compositions for parental administration are also sterile, substantially isotonic and made under GMP conditions.

Any of the above pharmaceutical compositions may in addition to the polypeptides of the invention comprise one or more further antimicrobial agents known in the art per se. For treating bacterial infections the pharmaceutical compositions may, in addition to the polypeptides of the invention, comprise antibiotics such as penicillins, cephalosporins, macrolides, fluoroquinolones, sulfonamides, tetracylcines or aminoglycosides.

For treating viral infections the pharmaceutical compositions may, in addition to the polypeptides of the invention, comprise antiviral nucleoside analogs such as aciclovir, ganciclovir, zidovudine (AZT) or didanosine or neuramidase inhibitors such as oseltamivir, peramivir or zanamivir. Similarly, for treating fungal infections the pharmaceutical compositions may, in addition to the polypeptides of the invention, comprise polyene antifungals, imidazoles, triazoles, allylamines, echinocandins or others, such as ciclopirox, flucytosine, griseofulvin, gentian violet, haloprogin, tolnaftate or undecylenic acid.

In yet another embodiment, the invention provides a method for treating a microbial infection in a patient in need thereof comprising providing to said patient a sufficient amount of a polypeptide or pharmaceutical composition as described herein.

As described above, a polypeptide according to the invention can be coupled to a suitable moiety such as a biotin or fluorescein label. These labelled mutated polypeptides can be used in a method for detecting or imaging microbial infections such as bacterial infections because they migrate to a site of microbial infection. Using a detector suitable for the used label attached to the polypeptide, it is possible to detect infection sites.

Methods for detecting microbial infections such as bacterial infections are therefore also provided by the invention. The method typically involves administering a labeled polypeptide to a patient infected with, or suspected of being infected with, some type of microbial organism. The polypeptide is any of the herein described polypeptides. Because the labeled polypeptide is capable of interacting with the infectious organism, it accumulates at the site of infection. It is possible to detect the accumulation of the polypeptide at site of infection using various detectors which are sensitive to the label that is attached to the polypeptide.

The polypeptide used can vary, but includes the polypeptides of the invention described herein.

The label utilized to label the polypeptide can vary widely; the label simply needs to be a molecule or macromolecule that is capable of generating a detectable signal and that can be attached to the polypeptide. Illustrative examples of such molecules include radioactive isotopes, fluorophors, chromophores, electron dense reagents, magnetic particles, enzymes, and ligands having a specific binding partner (e. g., biotin).

Similarly, the detector used to detect the label can be any device which is capable of detecting the signal generated by the label. For example, when the polypeptide is labeled with a radioactive isotope, the detector can include a gamma camera. Using such a camera it is possible to obtain images of the site of infection which can be utilized in various research, diagnostic and therapeutic applications.

The invention will be explained in more detail in the following, nonlimiting examples.

### Experimental part

### Materials and methods

### Example 1

### Peptides

Peptides designated Pep001-Pep096 were commercially obtained from Pepscan (Lelystad, Netherlands). The peptides were synthesized at 1 µmol per peptide in a 96 well format and used without further purification or analysis. After dissolving the peptides in 1 ml sterile MilliQ each, they were stored at 2-8° C for a maximum of 2 months.

Peptides designated MDxxx were synthesized at a 5 - 10 µmol scale with a purity of >95% at the Department of Medicinal Chemistry and Chemical Biology at the university of Utrecht (Utrecht, The Netherlands). Peptides designated JUPxxx were synthesized at a 50 µmol scale with >90% purity at Pepscan (Lelystad, Netherlands).

### Preparation of Mannonic acid derived hLF1-11 (Protocol Man-P01)

### Materials

- hLF1-11 (obtained from Peptisyntha, Torrance (CA), USA) of >95% purity
- N-(3-Dimethylaminopropyl)-N'-ethylcarbodiimide hydrochloride (EDAC), Sigma E7750
- Tetrafluorphenol (TFP), Aldrich 19-678-9
- L-Mannonic acid γ-lactone, Sigma M2261
- Sodium-bicarbonate (NaHCO₃), Sigma S-6014
- Acetonitril (100%), Merck 100029
- N-hydroxybenzotriazole (HoBt), Fluka S4802
- HPLC grade water, JT Baker - 4218
- Acetic-acid VWR 20099290
- Borosilicate glass tubes
- 1.5 mL Polypropylene Eppendorf tubes

### Step I

(A) Dissolve 20 mg TFP in 250 ml acetonitril. Note: Prior to addition of TFP to acetonitril heat TFP stock 30 min at 37 °C.
(B) Dissolve 5 mg Mannonic-acid in 1 ml water.
(C) Transfer 10 mg EDAC to a glass tube.
(D) Dilute solution A 10x by adding 25 ml of solution A to 225 ml Acetonitril.
(E) Add 25 ml of solution B to C.
(F) Add solution D to E and incubate 1h by 37 °C

### Step II

(G) Transfer 1 mg hLF1-11 peptide to an eppendorf tube
(H) Dissolve 2 mg HoBt in 1 mL Acetonitril.
(I) Add 2ml of H to F
(J) While gentle vortexing dissolve hLF peptide (G) in (I). Incubate 45 min at 37 °C

### Step III

(K) Dissolve 14 mg NaHCO₃ in 1 mL water.
(L) Add 20 ml of K to solution J.
   Incubate 45 min by 37 °C

### Step IV

Stop the reaction by adding approximately 700 ml of 0.01% acetic acid to obtain a final peptide concentration of 1 mg/mL.

### Bacterial strains

Antimicrobial properties of the different peptides against a gram negative bacteria (*Escherichia coli* ATCC 035), a gram positive bacteria (Methicillin Resistant *Staphylococcus aureus* (MRSA) ATCC 2141) both obtained from the American Type Culture Collection (ATCC, Manassas (VA), USA) and Yeast (*Candia Albicans* Y01-19; clinical isolate of LUMC, Leiden, the Netherlands) were used for the MIC determination.

### Minimal Inhibitory Concentration (MIC)

Minimal inhibitory concentrations (MICs) were determined by microdilution method according to CLSI guidelines using RPMI1640 medium diluted 1:4 in 0.2 µM sterile filtered MilliQ and an bacterial inoculum of 5x10⁵ CFU/ml. Plates were read after 20 - 24 h incubation at 35° C in ambient air. MICs were recorded as the lowest concentration that inhibited visible growth.

### Example 2

### Peptides

Peptides designated MD041-xx were synthesized at a 5 -10 µmol scale with a purity of >95% at the Department of Medicinal Chemistry and Chemical Biology at the university of Utrecht (Utrecht, The Netherlands). The reference peptide hLF1-11 was obtained from Peptisyntha, Torrance (CA), USA at gram scale and was purified to >95% purity. Peptides were dissolved in water for injection at a concentration of 1 mg/ml and diluted in saline to a final concentration of 20 microgram/ml, ready for intravenous infusion.

### Animals

65 Swiss out bred (IcoCaw OF1) female mice, 6-8 weeks upon arrival were obtained from Charles River Nederland, the Netherlands. They were kept on a commercial diet with a 12 hour light/dark cycle. Before starting the experiment they were acclimatized for 5 days.

### Thigh muscle infection model

Mice were injected with 2x10⁶ MRSA bacteria, strain 2141 (obtained from the American Type Culture Collection (ATCC, Manassas (VA), USA)) in the right thigh muscle, followed one day later by an intravenous injection of the test compounds, or vehicle (saline) in the tail vein or the positive control (vancomycin) 2 dd 100 mg/kg subcutanous. Forty-eight hours after infection mice were sacrificed and the right thigh muscle was removed. Thigh muscles were weighed and homogenized using an Ultra-Turrax^{®} and dilutions of the homogenate were prepared in saline. Limiting dilutions were plated onto agar plates and two days later the number of MRSA 2141 CFU (colony forming unit) were determined for each individual mouse to determine the outgrowth.

### Experimental part

### Results

### Examples 1

Table I shows peptides Pep002 to Pep096 which sequences have been derived from hLF1-11 (=reference peptide Pep001) by various amino acid deletions and amino acid. Table II shows another set of peptides (designated MDxxx, JUPxxx or MhLF), manufactured by different manufacturers using the same method and which sequence has been derived from hLF1-11 (=reference peptide MD013) by various amino acid deletions, amino acid substitution and/or additions of chemical groups to the peptides. Both tables show the MIC values of the various peptides for Methicillin Resistant *Staphylococcus aureus* (MRSA), *Escherichia coli* and/or *Candida albicans*. MIC values that divert not more than 1 dilution from that of the reference peptide (Pep001 for Pep002 to Pep096 and MD012 for all MDxxx, JUPxxx peptides and MhLF) are considered to be not significant different and the respective peptide to have equal antimicrobial activity. These MIC values as well as the corresponding modifications relative to the reference sequence have been highlighted in gray. As shown in Table I and II, several deletions, switches and additions do not influence antimicrobial potency of the peptide significantly whereas as others (e.g. switches that introduce negatively charged amino acids) completely abolish antimicrobial activity of the peptide. For a complete list of peptides that show similar activity compared to the reference peptide, please refer to Table I and II.

### Example 2

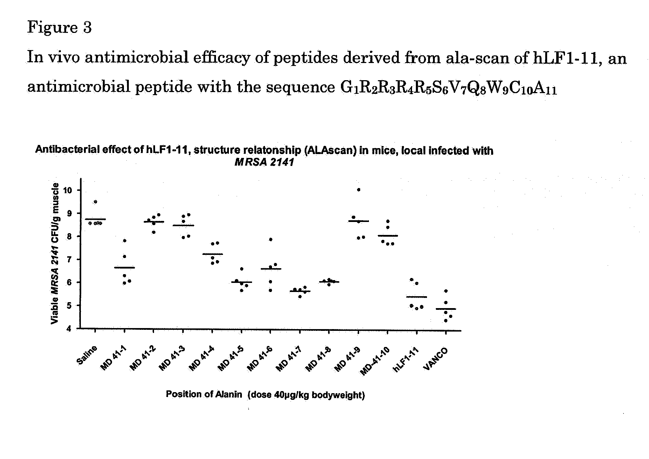

Figure 3 shows the effect on antimicrobial activity *in vivo* of compounds derived from an antimicrobial peptide with the sequence G₁R₂R₃R₄R₅S₆V₇Q₈W₉C₁₀A₁₁ using a single amino acid switch using alanine (a method known in the art as "alascan"). The peptides are designated MD41-1, where 1 stands for G₁ to A switch, MD41-2, where 2 stands for R₂ to A switch, and so on. As can be seen in Figure 3, positions 1, 4, 5, 6, 7 and 8 can be switched using alanine without loosing antimicrobial activity. The amino acid residues G₁, R₄, R₅ ,S₆,V₇, Q₈ are therefore considered to be non-crucial for *in vivo* antimicrobial activity.

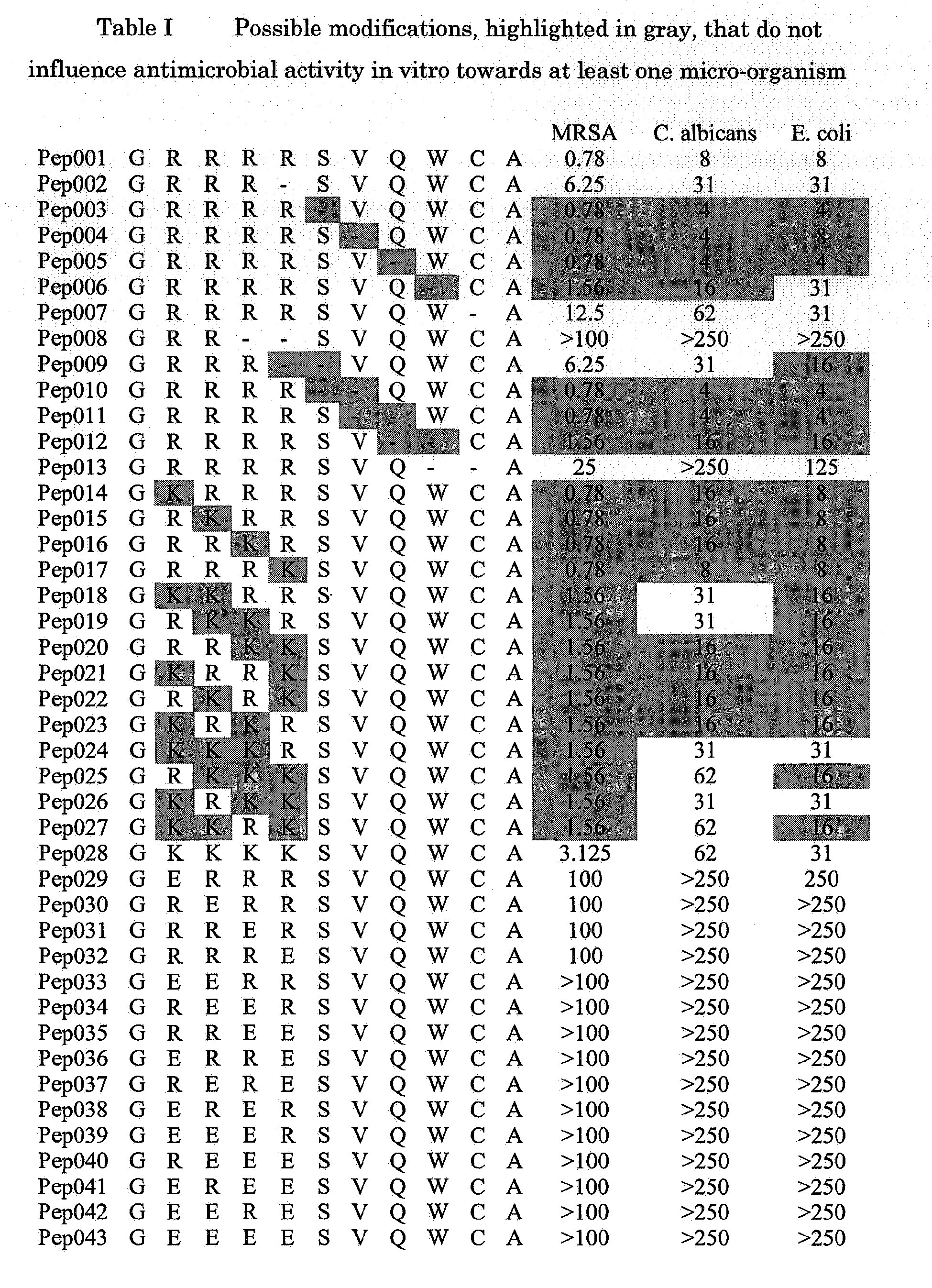

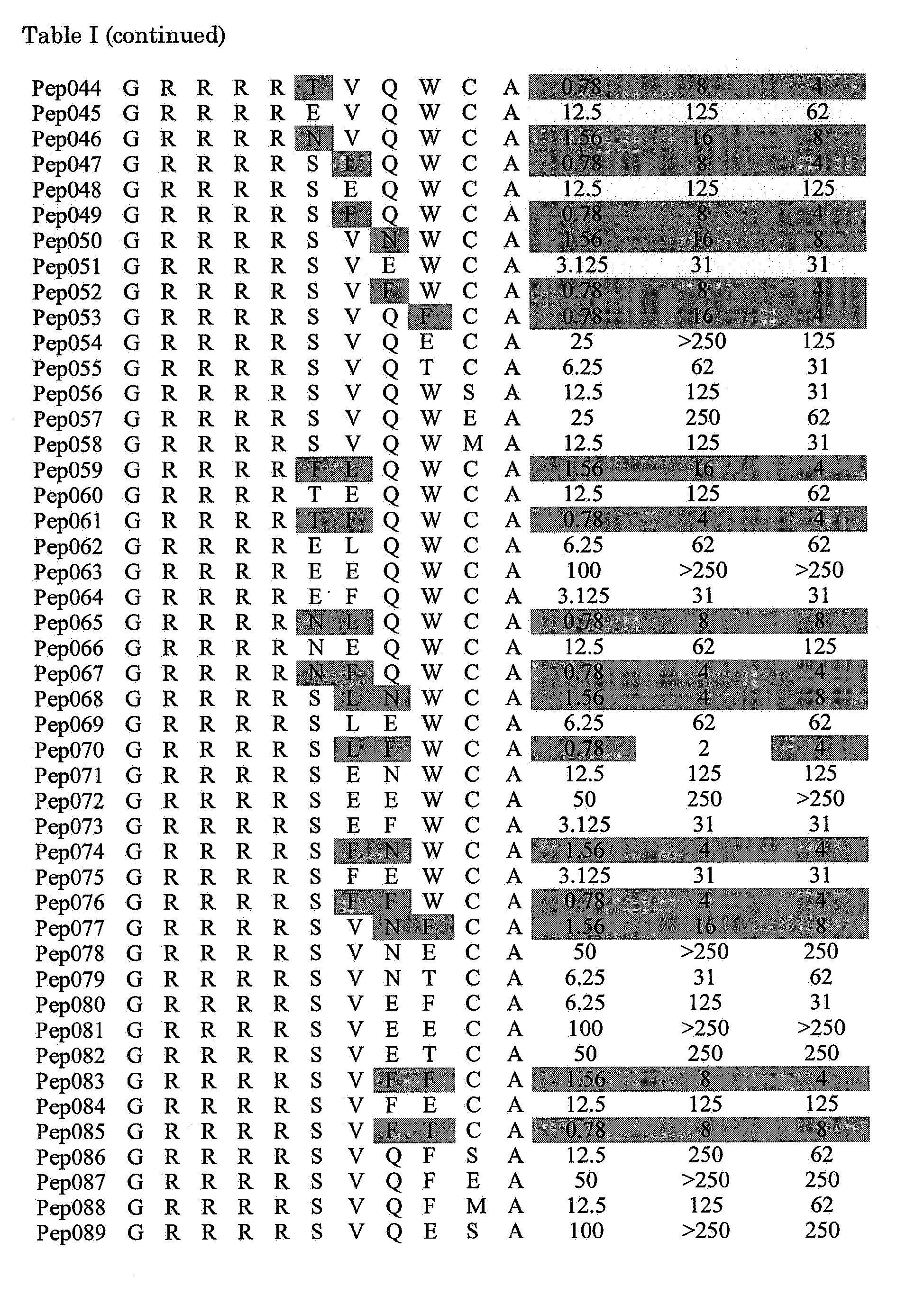

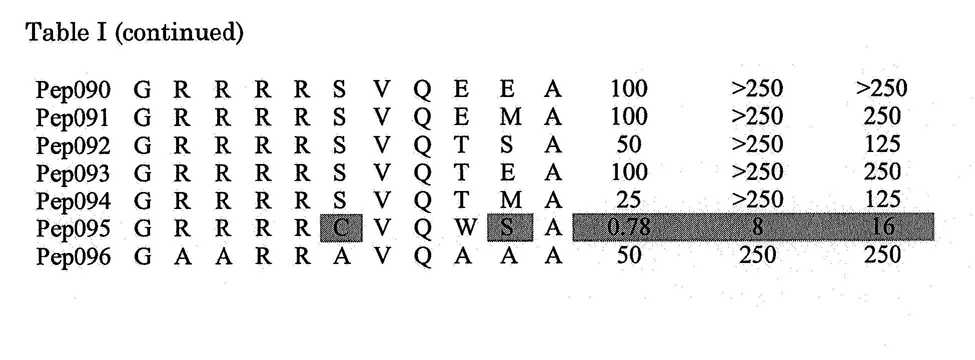

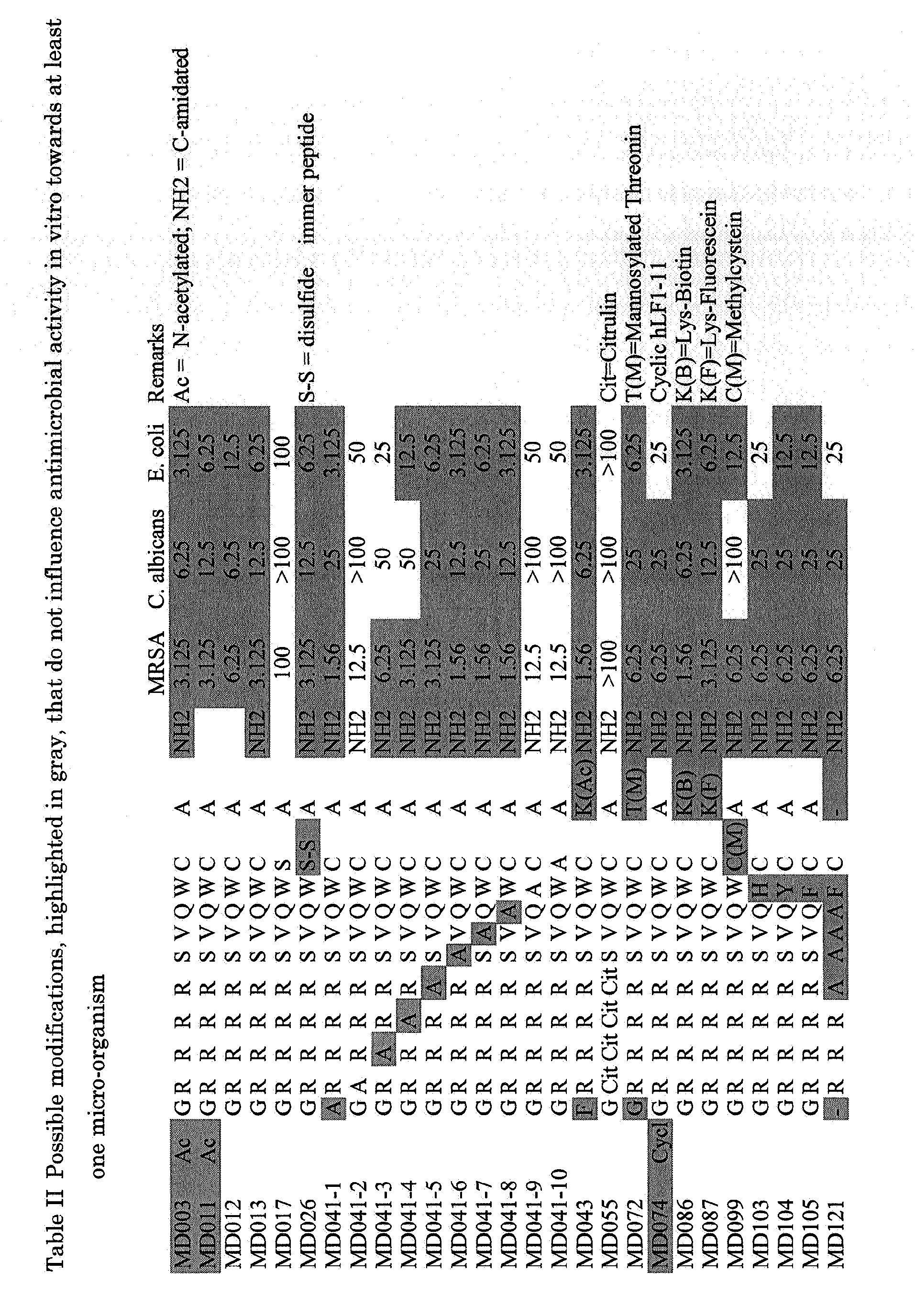

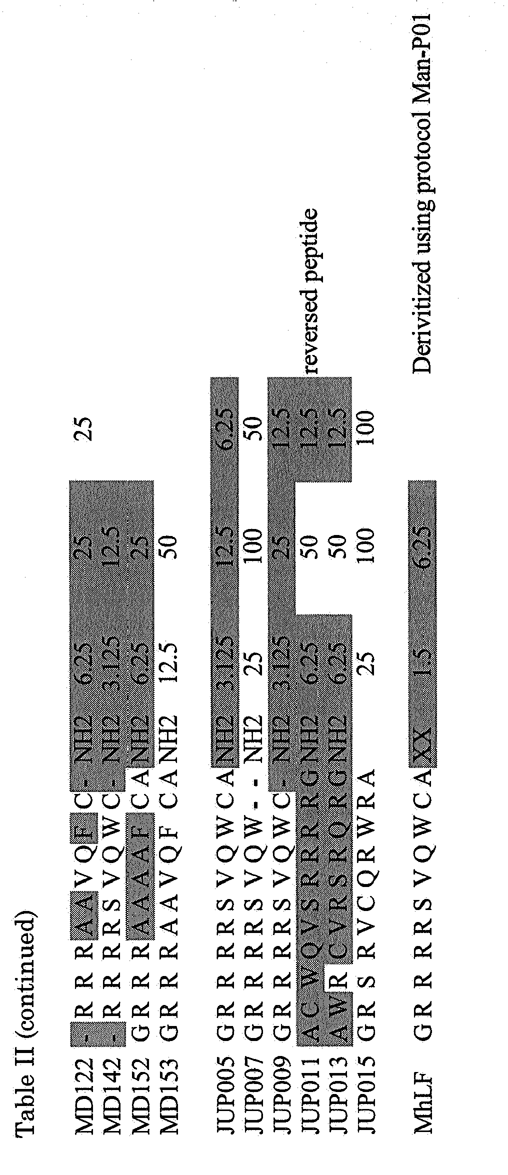

### Description of Figures

**Figure 1**
   Sequence of human lactoferrin (hLF; accession# EAW64767, NCBI GenPept)
**Figure 2**
   Structural formulas of hLF1-11 derivatives MD72, MD86 and MD87. MD72Mannosylated hLF1-11 (MW = 1565.76)
   MD86 Biotinylated hLF1-11 (Ala₁₁ = Lys₁₁) (MW = 1656.98)
   MD87 Fluorescein coupled hLF1-11 (Ala₁₁ = Lys₁₁) (MW = 1788.99)
**Figure 3**
   In vivo antimicrobial efficacy of peptides derived from ala-scan of hLF1-11, an antimicrobial peptide with the sequence G₁R₂R₃R₄R₅S₆V₇Q₈W₉C₁₀A₁₁ Groups of mice (n=5 each) were injected intramuscularly with 2 x 10⁶ MRSA, strain 2141 at day 0. At day 1 each group received either hLF1-11 (GRRRRSVQWCA), saline or one of the MD41-xx peptides i.v. (xx stands for position of amino acid in hLF sequence that is substituted for alanine). All peptides were given in a dosage of 40 µg/kg whereas the positive control (vanco) group received 2 s.c. injections at 20 hours and 28 hours after infection at a dose of 100 mg/kg. Each dot represents outgrowth of bacteria from a single mouse, 48 hours after infection and is a measure for antimicrobial infection of the tested compounds.

## Claims

1. A polypeptide comprising a mutant of the amino acid sequence RRRRSVQWC, which mutation comprising polypeptide has comparable antimicrobial activity against at least one micro-organism if compared to a reference polypeptide comprising the amino acid sequence RRRRSVQWC and wherein said mutant is:
• a deletion mutant in which at least one of the amino acids S, V, Q or W is deleted;
• a deletion mutant in which one R and at least one of the amino acids S, V, Q or W is deleted;
• a substitution mutant in which S, V or Q are substituted with a conservative or non-conservative amino acid, provided that (i) the substitute is not a negatively charged amino acid and (ii) S is not substituted with an N;
• a double substitution mutant in which SV, VQ or SQ are substituted with conservative or non-conservative amino acids, provided that none of the substitutes is a negatively charged amino acid;
• a triple substitution mutant in which SVQ are substituted with conservative or non-conservative amino acids, provided that none of the substitutes is a negatively charged amino acid;
• a substitution mutant in which W is replaced by another aromatic amino acid;
• a double substitution mutant in which Q or S is substituted by an aromatic amino acid and W is substituted by a neutral amino acid;
• a scrambled mutant in which C is switched with an amino acid in the SVQW sequence;
• a substitution mutant in which at least one but no more than three of the R's are substituted by another positively charged amino acid;
• a substitution mutant in which the second, third or fourth R is substituted by an A;
• any combination thereof.

2. A polypeptide according to claim 1, wherein said mutant is a deletion mutant in which S, V, Q or W is deleted or a deletion mutant in which RS, SV, VQ or QW are deleted.

3. A polypeptide according to claim 1 or 2, wherein said mutant is a double substitution mutant in which SV or VQ are substituted with conservative or non-conservative amino acids, provided that none of the substitutes is a negatively charged amino acid.

4. A polypeptide according to any one of claims 1 to 3, wherein said mutant is a double substitution mutant in which Q is substituted by an aromatic amino acid and W is substituted by a neutral amino acid.

5. A polypeptide according to any one of claims 1 to 4, wherein said mutant is a scrambled mutant in which S and C have changed places.

6. A polypeptide according to any one of claims 1 to 5, wherein said mutant is a double substitution mutant in which two R's are substituted by two other positively charged amino acid, preferably KK.

7. A polypeptide according to any one of claims 1 to 6, wherein said mutant is a triple substitution mutant in which three R's are substituted by three other positively charged amino acid, preferably KKK.

8. A polypeptide according to any one of claims 1 to 7, wherein the mutated amino acid sequence RRRRSVQWC is linear.

9. A polypeptide according to any one of claims 1 to 8, wherein said polypeptide has a length of 5 to 1000 amino acids.

10. A polypeptide according to claim 9, wherein said polypeptide has a length of 9, 10 or 11 amino acids.

11. A polypeptide according to any one of claims 1 to 10, further comprising a second moiety.

12. A polypeptide according to claim 11, wherein said second moiety is a protein.

13. A polypeptide according to claim 11 or 12, wherein said second moiety is a hormone.

14. A polypeptide according to claim 13, wherein said hormone is a pheromone.

15. A polypeptide according to claim 11, wherein said second moiety is a sugar group.

16. A polypeptide according to 15, wherein said sugar group is mannose.

17. A polypeptide according to claim 16, wherein said mannose is coupled to said polypeptide via a chemical coupling.

18. A polypeptide according to claim 11, wherein said second moiety is a lipid.

19. Use of a polypeptide according to any one of claims 1 to 18 for use as a medicament.

20. A pharmaceutical composition comprising a polypeptide according to any one of claims 1 to 18 and a pharmaceutical acceptable carrier, diluent or excipient.

21. A pharmaceutical composition according to claim 20, further comprising a conventional antimicrobial agent.

22. Use of a polypeptide according to any one of claims 1 to 18 for the manufacture of a medicament for the treatment or prophylaxis of microbial infections.

23. Use according to claim 22, wherein said microbe is a bacterium, a fungus, a virus or a parasite.
